# EUROPEAN PATENT APPLICATION

(11) **EP 4 066 894 A1**
(43) Date of publication of application: **05.10.2022**
(21) Application number: 20892455.5
(22) Date of filing: 18.11.2020
(51) Int. Cl.: A61P 3/10, C12N 5/077, C12Q 1/02, A61K 38/18, G01N 33/15, G01N 33/48, G01N 33/50, A61K 35/35, A61K 31/352, A61K 31/4439

(54) **BROWN ADIPOCYTE PRODUCTION METHOD**

(30) Priority: 25.11.2019 JP 2019211990
(71) Applicant: Kataoka Corporation, Kyoto-shi, Kyoto 601-8203 (JP); Kyoto Prefectural Public University Corporation, Kyoto-shi, Kyoto 602-8566 (JP)
(72) Inventor: DAI Ping, Kyoto-shi Kyoto 602-8566 (JP); TAKEDA Yukimasa, Kyoto-shi Kyoto 602-8566 (JP); HARADA Yoshinori, Kyoto-shi Kyoto 602-8566 (JP); MATSUMOTO Junichi, Kyoto-shi Kyoto 601-8203 (JP)
(74) Representative: Vossius & Partner Patentanwälte Rechtsanwälte mbB
(86) International application number: PCT/JP2020/042898
(87) International publication number: WO 2021/106697

(57) **Abstract**

The present invention is primarily directed to provide a new process capable of performing direct conversion of or induction from a somatic cell to brown adipocytes with low molecular weight compounds, without performing artificial gene transfer.

The present invention includes, for example, a process for producing brown adipocytes by direct differentiation induction from somatic cells, comprising a step of culturing a somatic cell in a serum-free differentiation induction medium in the presence of a selective PPARγ agonist and a cAMP inducer.

According to the present invention, direct conversion of or induction from somatic cells to brown adipocytes can be effectively performed without gene transfer. The brown adipocytes obtained by the present invention are useful as regenerative medicine, models of human brown adipocytes and human beige cells, and the like.

## Description

### TECHNICAL FIELD

### (Cross-Reference to Related Applications)

This application claims the benefit of Japanese Patent Application No. 2019-211990, filed November 25, 2019 with the Japanese Patent Office. The Japanese application is hereby incorporated by reference for all purposes as if the entirety of its application documents (specification, claims, drawings, abstract) were expressly set forth herein.

The present invention belongs to the technical field of regenerative medicine. The present invention relates in its technical field to a method for producing brown adipocytes from somatic cells by low molecular weight compounds, and to low molecular weight compound-induced brown adipocytes (ciBAs: chemical compound-induced Brown Adipocytes) produced by such a process. The present invention further relates to a composition for an induction medium which can be used for a method of producing said brown adipocytes.

### BACKGROUND OF THE INVENTION

In recent years, methods (direct reprogramming) for converting somatic cells, such as fibroblasts, directly into other cell types by low molecular weight compounds without gene transfer have been actively studied. For example, in Patent Document 1, several low molecular weight compounds such as an ALK5 inhibitor and an ALK6 inhibitor are combined, and in the presence thereof, somatic cells, particularly human fibroblasts, are cultured, and are directly converted into brown adipocytes and the like. As in the invention of Patent Document 1, it is very beneficial if conventional fibroblasts can be directly converted into other cell types such as brown adipocytes with a low molecular weight compound which is easy to obtain. For example, autologous fibroblasts can be conveniently used to produce other autologous cell types, thereby increasing their applications in regenerative medicine. In addition, cells for the development of new pharmaceuticals can be easily produced as experimental materials.

In Non-Patent Document 1, as in Patent Document 1, human skin fibroblasts are cultured in the presence of a combination of several low molecular weight compounds and brown adipocytes are directly induced from such fibroblasts. Specifically, in the invention of Non-Patent Document 1, human skin fibroblasts are cultured in the presence of low molecular weight compounds such as SB431542 (ALK5 inhibitor), LDN193189 (ALK2/3 inhibitor), dorsomorphin (ALK2/3/6 inhibitor), forskolin (cAMP inducer), and rosiglitazone (PPARγ agonist), and brown adipocytes are directly induced from such fibroblasts. The combination of said low molecular weight compounds is also disclosed in Patent Document 1.

The differentiation induction from somatic cells to brown adipocytes and the like as described above is always performed by culturing under a medium containing various other compounds, nutrients and the like. The medium can include, for example, conventional mediums such as MEMs (minimal essential medium), DMEM (Dulbecco's Modified Eagle's Medium), DMEM/F12, or a modified medium thereof, and these are commercially available. Furthermore, in general, appropriate components (serum, proteins, amino acids, saccharides, vitamins, fatty acids, antibiotics, and the like) are added to these medium in consideration of cells to be induced, and the cell differentiation is carried out. Since serum such as fetal bovine serum (FBS) contains components necessary for cell growth such as hormones, growth factors, and adhesion factors, it is often carried out to induce differentiation by adding serum In the inventions of Patent Document 1 and Non-Patent Document 1, differentiation induction is carried out under a medium to which FBS is added.

### PRIOR ART

### PATENT DOCUMENT

Patent Document 1: WO 2018/062269

### NON-PATENT DOCUMENT

Non-Patent Document 1: Takeda Y., Harada Y., Yoshikawa T., and Dai P., Sci. Rep., 7, 4304 (2017)

### SUMMARY OF THE INVENTION

### PROBLEM TO BE SOLVED BY THE INVENTION

The present invention is primarily directed to provide a new process capable of performing direct conversion of or induction from a somatic cell to brown adipocytes with low molecular weight compounds, without performing artificial gene transfer. It is also an object of the present invention to provide a new composition for culturing somatic cells in the presence of low molecular weight compounds and producing brown adipocytes by direct conversion.

### MEANS FOR SOLVING THE PROBLEMS

As a result of intensive studies, the present inventors have found that the above problem can be solved by a combination of certain low molecular weight compounds in serum-free condition, and have completed the present invention.

The present invention, for example, can include the following embodiments.
[1] A process for producing brown adipocytes by direct differentiation induction from somatic cells, comprising a step of culturing a somatic cell in a serum-free differentiation induction medium in the presence of a selective PPARγ agonist and a cAMP inducer.
[2] The process for producing brown adipocytes according to the [1] mentioned above, wherein the step is a step of culturing a somatic cell in the further presence of BMP7
[3] The process for producing brown adipocytes according to the [1] or [2] mentioned above, wherein the step is a step of culturing a somatic cell in the absence of an ALK2/3 inhibitor and an ALK6 inhibitor.
[4] The process for producing brown adipocytes according to any one of the [1] to [3] mentioned above, wherein the step is a step of culturing a somatic cell in the further absence of an ALK5 inhibitor.
[5] The process for producing brown adipocytes according to any one of the [1] to [4] mentioned above, wherein the selective PPARγ agonist is rosiglitazone or the cAMP inducer is forskolin.
[6] The process for producing brown adipocytes according to any one of the [1] to [5] mentioned above, wherein the somatic cell is a fibroblast.
[7] A brown adipocyte, produced by the process according to any one of the [1] to [6] mentioned above.
[8] A differentiation induction composition for producing brown adipocytes by directly inducing differentiation from somatic cells, comprising a selective PPARγ agonist and a cAMP inducer and being serum-free.
[9] The differentiation induction composition according to the [8] mentioned above, further comprising BMP7.
[10] The differentiation induction composition according to the [8] or [9] mentioned above, wherein the selective PPARγ agonist is rosiglitazone or the cAMP inducer is forskolin.
[11] The differentiation induction composition according to any one of the [8] to [10] mentioned above, wherein the somatic cell is a fibroblast.
[12] A method of screening an agent acting on brown adipocytes, comprising using the brown adipocyte produced by the process according to any one of the [1] to [6] mentioned above, or the brown adipocyte according to the [7] mentioned above.
[13] A method of evaluating the degree of enhancement of browning tendency by a test substance in the brown adipocyte produced by the process according to any one of the [1] to [6] mentioned above or the cell under production thereof, or the brown adipocyte according to the [7] mentioned above, comprising an addition of the test substance to the cells.

### EFFECT OF THE INVENTION

According to the present invention, direct conversion or direct differentiation induction from somatic cells to brown adipocytes can be effectively performed in serum-free condition, without gene transfer. The brown adipocytes obtained by the present invention are useful for regenerative medicine, a human brown adipocyte model, and the like.

### BRIEF DESCRIPTION OF THE DRAWINGS

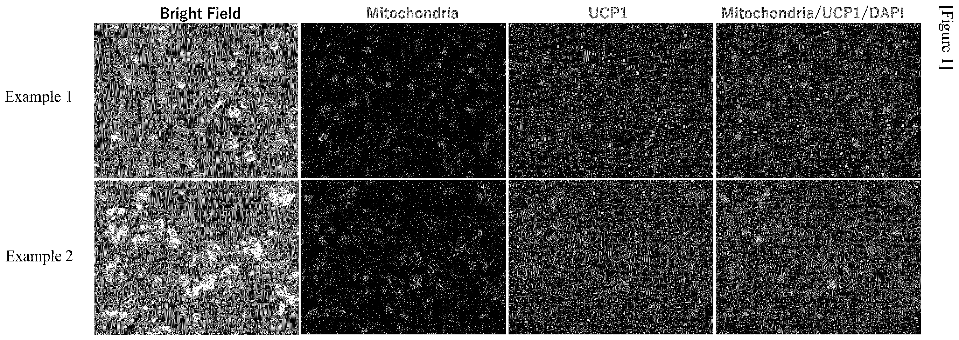

[Figure 1] Photographs of the cultured cells, and photographs of the cells in which mitochondria (red), UCP1 protein (green) and cell nucleus (blue) were immuno-stained. Since Figure 1 is monochrome, the colors red, green, and blue are not displayed, but the colors are displayed in the original photograph of Figure 1. Top four figures represent the results obtained with the combination of the compounds of Example 1, and bottom four figures represent the results obtained with the combination of the compounds of Example 2.

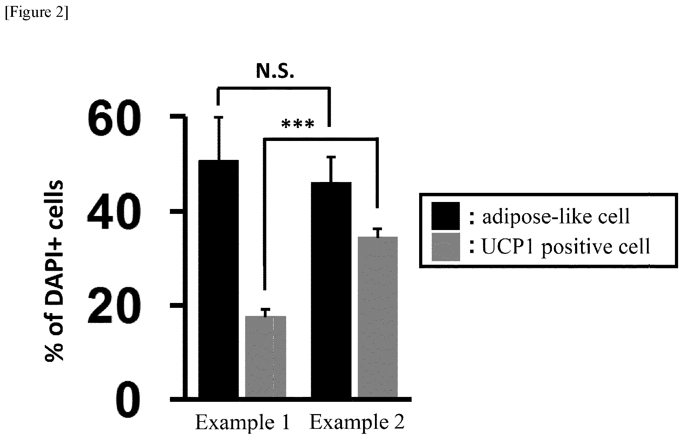

[Figure 2] The rates of adipose-like cells and UCP1 positive cells are represented. The left two columns represent the results obtained with the combination of the compounds of Example 1 and the right two columns represent the results obtained with the combination of the compounds of Example 2. In the results of each Example, the left-hand column represents the rate of adipose-like cells and the right-hand column represents the rate of UCP1 positive cells. The vertical axis shows the rate (%) of these cells relative to the cell nuclear stain (DAPI) positive cells.

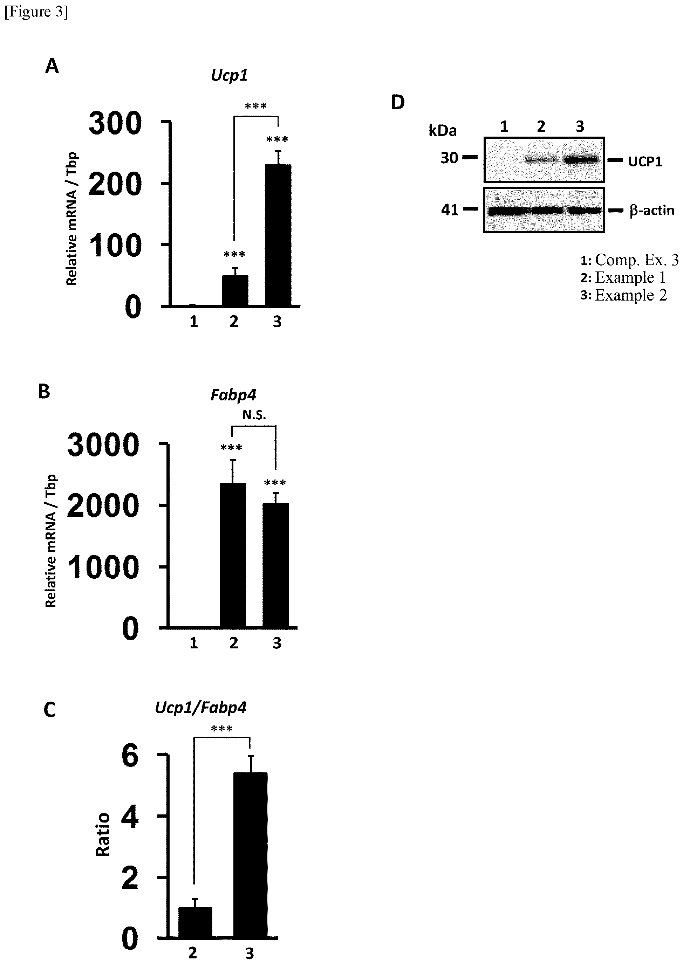

[Figure 3] Figure A shows the expression levels of Ucp1 genes, Figure B shows the expression levels of Fabp4 genes, Figure C shows the ratio of Ucp1/Fabp4, and Figure D is an electrophoretic photograph in which UCP1 proteins were quantified. Symbol 1 denotes Comparative Example 3 (control), Symbol 2 denotes Example 1, and Symbol 3 denotes Example 2. The vertical axis represents the mRNA amount of the gene relative to that of the internal standard gene (Tbp).

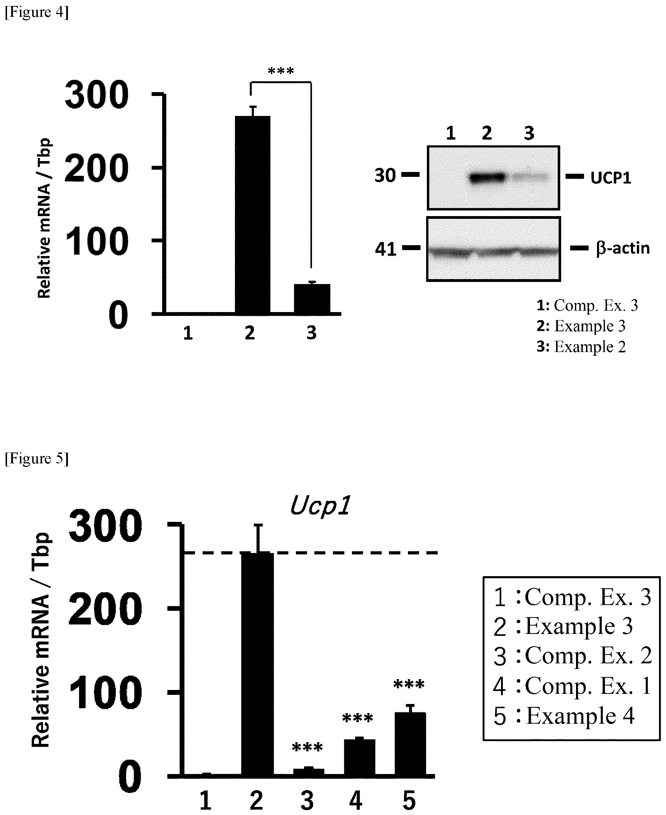

[Figure 4] The left-hand figure shows the expression levels of Ucp1 genes, and the right-hand figure shows electrophoresis photographs in which UCP1 proteins were quantified. Symbol 1 denotes Comparative Example 3 (control), Symbol 2 denotes Example 3, and Symbol 3 denotes Example 2. The vertical axis represents the mRNA amount of the gene relative to that of the internal standard gene (Tbp).
[Figure 5] Expression levels of Ucp1 genes are represented. Symbol 1 denotes Comparative Example 3 (control), Symbol 2 denotes Example 3, Symbol 3 denotes Comparative Example 2, Symbol 4 denotes Comparative Example 1, and Symbol 5 denotes Example 4. The vertical axis represents the mRNA amount of the gene relative to that of the internal standard gene (Tbp).

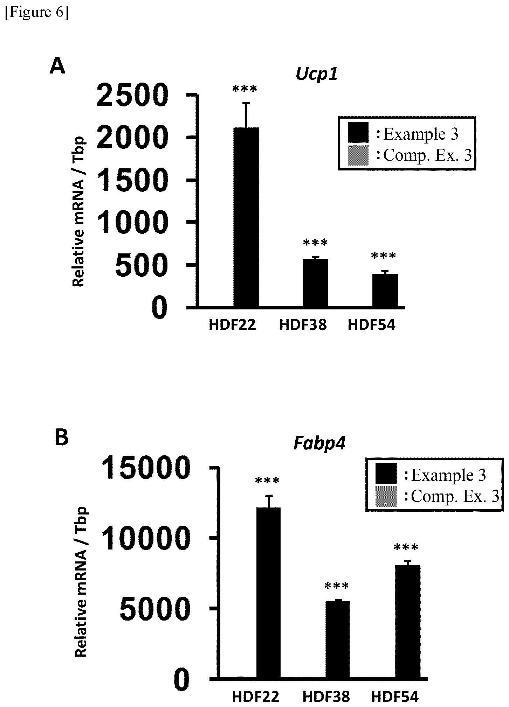

[Figure 6] Figure A shows the expression levels of Ucp1 genes, and Figure B shows the expression levels of Fabp4 genes. In the results of skin fibroblasts derived from different human samples, each left-hand column represents the result obtained with no compound (Comparative Example 3), and each right-hand column represents the result obtained with the combination of compounds of Example 3. The vertical axis represents the mRNA amount of the gene relative to that of the internal standard gene (Tbp).

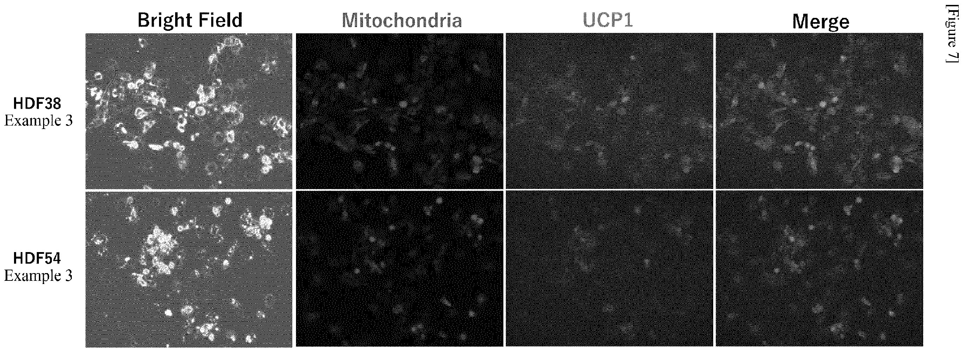

[Figure 7] Photographs of the cultured cells, and photographs of the cells in which mitochondria (red) and UCP1 protein (green) were immuno-stained, and photographs in which cell nuclei (blue) were stained and merged images thereof. Since Figure 7 is monochrome, the colors green, red, and blue are not displayed, but the colors are displayed in the original photograph of Figure 7.

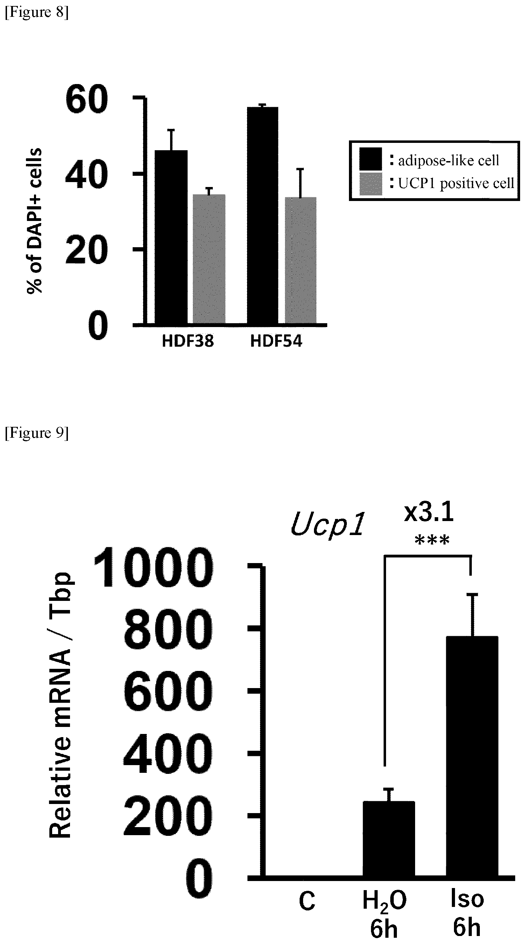

[Figure 8] The rates of adipose-like cells and UCP1 positive cells are represented. In the result of each human fibroblast, each left-hand column represents the rate of adipose-like cells and each right-hand column represents the rate of UCP1 positive cells.
[Figure 9] Activation of Ucp1 genes expression is shown. C shows the expression levels of control cells (no addition of compound, Comparative Example 3). H₂O6h shows the result obtained after 6 hours of culture with the addition of control H₂O (water) of the cells which had been induced with the compound combinations of Example 3, followed by further culturing in a serum-free brown adipocyte maintenance medium (SFBAMaM). Iso6h shows the result obtained after 6 hours of culture with the addition of Isoproterenol, a β-adrenergic receptor agonist, instead of H₂O aforementioned. The vertical axis represents the mRNA amount of the gene relative to that of the internal standard gene (Tbp).

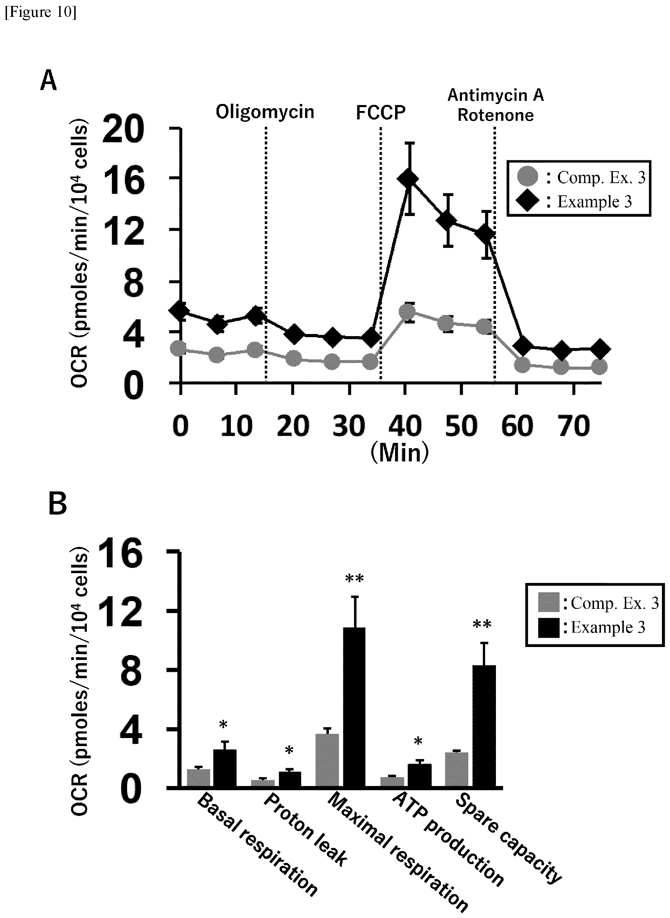

[Figure 10] Figure A shows the change of the oxygen consumption rate. Grey circles represent the result in control cells (no addition of compound, Comparative Example 3), and black diamonds represent the result in brown adipocytes derived from fibroblasts with the combination of compounds of Example 3. In the figure, the dotted line indicates that oligomycin, FCCP or antimycin A/rotenone was added. The horizontal axis represents time (minutes). Figure B shows the oxygen consumption rate corresponding to each parameter on the horizontal axis. In the result of each parameter, each left-hand column represents the result obtained with no compound (Comparative Example 3), and each right-hand column represents the result obtained with the combination of compounds of Example 3. The vertical axis represents the oxygen-consumption rate OCR (picomole/minute/10⁴ cells).

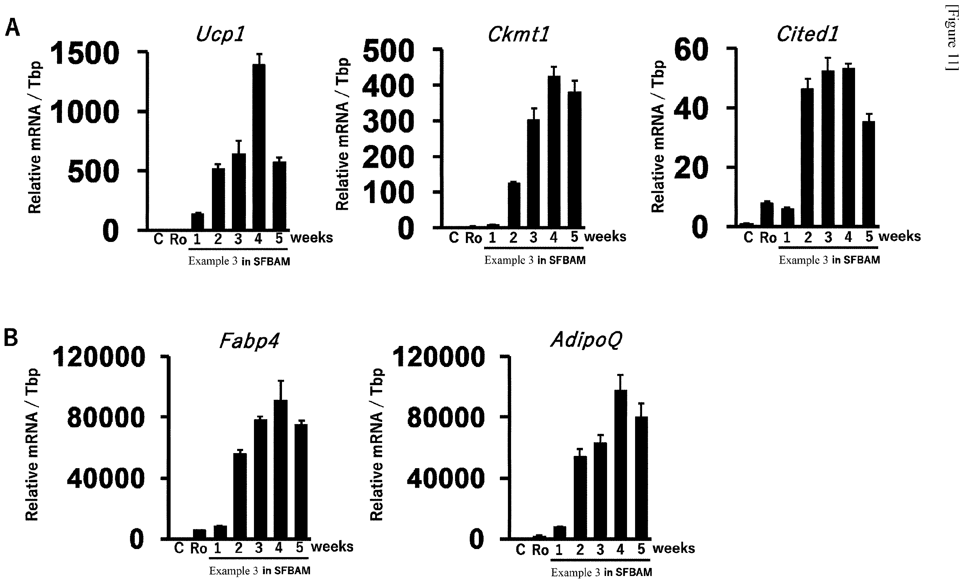

[Figure 11] Expression levels of the gene are represented. In each figure, the vertical axis represents the mRNA amount of the gene relative to that of the internal standard gene (Tbp). Numerals on the horizontal axis indicate time (weeks).

### MODE FOR CARRYING OUT THE PRESENT INVENTION

Hereinafter, the present invention will be described in detail.

### 1 Method for producing brown adipocytes

The process for producing brown adipocytes according to the present invention (hereinafter referred to as the "present invention process") is characterized by comprising a step of culturing a somatic cell in a serum-free differentiation induction medium in the presence of a selective PPARγ agonist and a cAMP inducer.

Here, "serum-free" refers to the state of being substantially free of unadjusted or unpurified serum, and in the present invention, even if a differentiation induction medium is contaminated with a purified component derived from blood or animal tissue, the medium is referred to as a serum-free differentiation induction medium, so long as it does not substantially contain an unadjusted or unpurified serum

The "brown adipocytes" (ciBAs) produced by the present invention process are equivalent to so-called brown adipocytes, which have many mitochondria and many fat droplets and express UCP1(uncoupling protein-1). Thus, it can be referred to as simply brown adipocytes or brown fat-like cells. It can also be a model for so-called human beige cells and bright cells.

It is preferable that the above step in the present invention process is a step of culturing a somatic cell in the further presence of BMP7.

In each of the selective PPARγ agonist and the cAMP inducer, either one kind or two or more kinds in combination may be used.

### 1.1 Somatic cell

Cells of an organism can be classified into somatic and germ cells. Any somatic cell can be used as a starting material in the present invention process. The somatic cell is not particularly limited, and may be either a primary cell taken from a living body or a cell from an established cell line. Somatic cells at various stages of differentiation, e.g., terminally differentiated somatic cells (e.g., fibroblasts, umbilical vein endothelial cells (HUVEC), hepatic cells (Hepatocytes), bile duct cells (Biliary cells), pancreatic alpha cells (Pancreatic α cells), pancreatic acinar cells (Acinar cells), pancreatic ductal glandular cells (Ductal cells), small intestinal crypt cells (Intestinal crypt cells), etc.), somatic cells on the way to terminal differentiation (e.g., mesenchymal stem cells, neural stem cells, endodermal progenitor cells, etc.), or such somatic cells like iPS cells that have been initialized to acquire pluripotency and somatic cells that have differentiated or are in the process of differentiation from iPS cells, can be used in the present invention process. The somatic cells that can be used in the present invention process include any somatic cells, for example, cells of the hematopoietic system (various lymphocytes, macrophages, dendritic cells, bone marrow cells, etc.), cells derived from organs (hepatocytes, splenocytes, pancreatic cells, kidney cells, lung cells, etc.), cells of the muscle tissue system (skeletal muscle cells, smooth muscle cells, myoblasts, cardiomyocytes, etc.), fibroblasts, neurons, osteoblasts, chondrocytes, endothelial cells, interstitial cells, adipocytes (white adipocytes, etc.), embryonic stem cells (ES cells), etc. The present invention process can also be applied to the precursor cells of these cells and cancer cells. Preferably, fibroblasts can be used.

Fibroblasts that can be used in the present invention are not particularly limited, and examples of the fibroblasts can include skin fibroblasts (Dermal Fibroblasts), aortic outer membrane fibroblasts (Adventitial Fibroblasts), cardiac fibroblasts (Cardiac Fibroblasts), pulmonary fibroblasts (Pulmonary Fibroblasts), uterine fibroblasts (Uterine Fibroblasts), chorionic mesenchymal fibroblasts (Villous Mesenchymal Fibroblasts), all of which are primary cell components constituting connective tissues in various tissues and organs, and are cells producing collagen fibers.

Examples of the source of the above-mentioned somatic cells include, but are not limited to, humans, mammals other than humans, and animals other than mammals (birds, reptiles, amphibians, fishes, and the like). As a source of somatic cells, humans and mammals other than humans are preferred, and humans are particularly preferred. When brown adipocytes are produced by the present invention process for the purpose of administration to humans, preferably, somatic cells harvested from a donor having a matched or similar type of histocompatibility antigen to the recipient can be used. Somatic cells harvested from the recipient itself may be used for the production of brown adipocytes by the present invention process.

### 1.2 Selective PPARγ agonist

PPARγ (Peroxisome proliferator-activated Receptor γ) is one of the proteins belonging to the nuclear receptor superfamily and is a transcription factor that controls gene expression by forming heterodimers with retinoid X receptors (RXRs) and binding to the promoter regions of target genes. There are three subtypes of PPARs: α, δ (β), and γ, and the present invention uses an agonist that selectively acts on γ.

The term "in the presence of a selective PPARγ agonist" refers to a culture condition under which an agonist action can be selectively exerted on PPARγ, and the means is not particularly limited, and a compound which selectively exerts an agonist action on PPARγ can be usually utilized.

Although not particularly limited in the present invention, examples of the selective PPARγ agonist include the following compounds or salts thereof. Preference is given to selective PPARγ agonists of the thiazolidine system, in particular rosiglitazone, pioglitazone, and troglitazone.
Rosiglitazone (CAS No.: 122320-73-4)

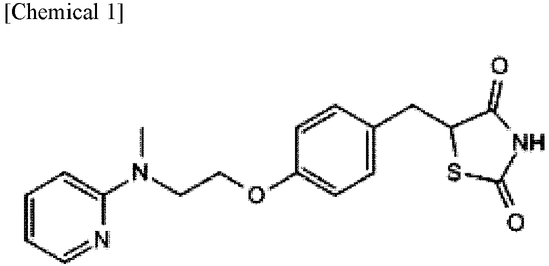

Rosiglitazone Maleate (CAS No.: 155141-29-0)
Ciglitazone (CAS No.: 74772-77-3)
Edaglitazone (CAS No.: 213411-83-7)
GW1929 Hydrochloride (CAS No.: 1217466-21-1)
nTZDpa (CAS No.: 118414-59-8)
Pioglitazone Hydrochloride (CAS No.: 112529-15-4)
S26948 (CAS No.: 353280-43-0)
Troglitazone (CAS No.: 97322-87-7)
Baraglitazone (CAS No.: 199113-98-9)
Rivoglitazone (CAS No.: 185428-18-6)

The concentration of the selective PPARγ agonist varies depending on the kind of the agonist, the somatic cell used, and the like, but is not particularly limited and may be appropriately determined, and may be used in a range of, for example, 0.1 µmol/L to 20 µmol/L, preferably 0.5 µmol/L to 5 µmol/L.

### 1.3 cAMP inducer

cAMP (cyclic adenosine monophosphate) is a second messenger that is involved in a variety of intracellular signaling events. The cAMP is produced intracellularly by cyclization of adenosine triphosphate (ATP) by adenylyl cyclase (adenylate cyclase).

The term "in the presence of a cAMP inducer" refers to a culture condition capable of inducing cAMP, and the means thereof is not particularly limited, and for example, any means capable of increasing the intracellular cAMP level can be used. Substances that can induce cAMP by acting directly on adenylyl cyclase, an enzyme involved in the production of cAMP, substances that can promote the expression of adenylyl cyclase, and substances that inhibit phosphodiesterase, an enzyme that degrades cAMP, can be used as measures to increase intracellular cAMP concentrations. It is also possible to use dibutyryl cAMP, 8-bromo-cAMP, and the like, which are a structural analogue of cAMP having the same effect as cAMP in cells.

Although not particularly limited in the present invention, examples of the cAMP inducer (adenylate cyclase activator) include forskolin (CAS No.: 66575-29-9), and forskolin derivatives (e.g., JP 2002-348243) and the following compounds. Preferably, forskolin can be used.
Forskolin (CAS No.: 66428-89-5)

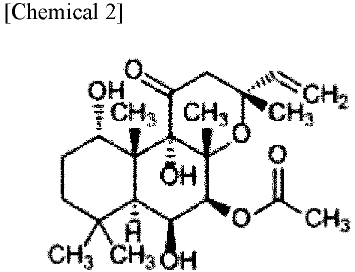

Isoproterenol (CAS No.: 7683-59-2)
NKH477 (CAS No.: 138605-00-2)
PACAP1-27 (CAS No.: 127317-03-7)
PACAP1-38 (CAS No.: 137061-48-4)

The concentration of the cAMP inducer varies depending on the kind of the inducer, the somatic cell used, and the like, but is not particularly limited and may be appropriately determined, and may be used in a range of, for example, 0.1 µmol/L to 100 µmol/L, preferably 0.5 µmol/L to 30 µmol/L.

### 1.4 BMP7

BMP (Bone Morphogenetic Protein: osteogenic proteins) 7 is one of the BMP families. Human BMP7 consists of 117 amino acid residues and activates the BMP signaling pathway by binding to cell surface receptors as a homodimer. Six types from BMP2 to BMP7 are classified as TGFb superfamilies, and are respectively encoded by distinct genes. In the present invention, either of a BMP7 derived from a human or a BMP7 derived from an animal may be used, and a recombinant thereof may be used. Among them, it is preferable to use a BMP7 derived from a human.

The concentration of the BMP7 varies depending on the kind of the BMP7, the somatic cell used, and the like, but is not particularly limited and may be appropriately determined, and may be used in a range of, for example, 0.1 ng/mL to 200 ng/mL, preferably 1 ng/mL to 50 ng/mL.

### 1.5 Preferred culture condition

Although not particularly limited in the present invention, in the above steps in the present invention process, it is more preferable to culture a somatic cell in the absence of an ALK2/3 inhibitor (an agent that inhibits both ALK2 and 3) and an ALK6 inhibitor, to culture a somatic cell in the absence of an ALK5 inhibitor, or to culture a somatic cell in the absence of an ALK2/3 inhibitor, an ALK6 inhibitor, and an ALK5 inhibitor.

The absence of an ALK2/3 inhibitor, an ALK6 inhibitor, or an ALK5 inhibitor means that they are substantially absent, and it is intended to include not only the case where none of them are present at all, but also the case where they are present in such an amount that these effects are not exhibited.

ALK2 is a receptor serine/threonine kinase of ALK family members, located upstream of signaling pathways involving SMAD proteins, particularly SMAD 1/5/8. Endoglin reduces the motility of prostatic carcinoma cells through activation of ALK2-Smad1 pathway. ALK2 gene is a major gene involved in fibrodysplasia ossificans progressiva (FOP), an unusual autosomal dominant congenital disorder characterized by progressive heterotopic bone formation in muscle tissue.

ALK3 is a member of the transmembrane serine-threonine kinase family. ALK3 gene acts as a minor susceptibility gene in PTEN (phosphatase and tensin homologue deleted on chromosome 10) mutation-negative Cowden's disease. ALK3 trafficking plays key roles in the pathogenesis of FOPs and is also involved in human T-cell differentiation.

Specific ALK2/3 inhibitors can include, for example, LDN193189 (CAS No.: 1062368-24-4), dorsomorphine (CAS No.: 866405-64-3), K02288 (CAS No.: 1431985-92-0), LDN212854 (CAS No.: 1432597-26-6), LDN193189 hydrochloride (CAS No.: 1062368-62-0), ML347 (CAS No.: 1062368-49-3), and LDN214117 (CAS No.: 1627503-67-6).

ALK6 is known also as BMPR1B, and is a transmembrane serine/threonine kinase member in the bone morphogenetic protein (BMP) receptor members. The ALK6 is considerably similar to activin receptors ACVR1 and ACVR2, and involved mainly in osteogenesis in a cartilage and embryogenesis.

Specific ALK6 inhibitors can include, for example, dorsomorphine (CAS No.: 866405-64-3), and K02288 (CAS No.: 1431985-92-0).

ALK5 is a TGFβ receptor subfamily member also referred to as TGFβR1 (transforming growth factor β receptor 1). The ALK5 is a serine/threonine kinase that forms a heterodimeric complex with a type II TGFβ receptor in response to binding with TGFβ, and transmits TGFβ signals from a cell surface to a cytoplasm.

Specific ALK5 inhibitors can include, for example, SB431542 (CAS No.: 301836-41-9), A83-01 (CAS No.: 909910-43-6), Repsox (CAS No.: 446859-33-2), D4476 (CAS No.: 301836-43-1), LY364947 (CAS No.: 396129-53-6), SB525334 (CAS No.: 356559-20-1), and SD208 (CAS No.: 627536-09-8).

### 1.6 Somatic cell culture

Culturing of somatic cells in the present invention process can be carried out by a conventional method using a serum-free differentiation induction medium in the presence of a selective PPARγ agonist and a cAMP inducer described above, selecting a medium, a temperature, or other conditions depending on the kind of somatic cells to be used.

In the present invention process, brown adipocytes can be produced from somatic cells in one step by culturing the somatic cells in a serum-free differentiation induction medium containing a selective PPARγ agonist and a cAMP inducer described above.

Also, selection of somatic cells easy to culture as the somatic cells to be used allows conversion into brown adipocytes of the somatic cells of which the number has been previously increased to reach a substantially confluent state. Thus, the scaled-up production of brown adipocytes is also easy.

The basal differentiation induction medium or medium for subculturing somatic cells in practicing the present invention may be selected from known or commercially available media. For example, MEM (Eagle's Minimum Essential Medium), GMEM (Glasgow's Minimum Essential Medium), DMEM (Dulbecco's Modified Eagle's Medium), Ham's F-12, DMEM/F12, RPMI1640, IMDM (Iscov's Modified Dulbecco's Medium), all of which is a general basal medium, or a medium modified therewith can be used by adding appropriate components (serum, proteins, amino acids, saccharides, vitamins, fatty acids, antibiotics, and the like).

As a culture condition, a general condition of cell culture may be appropriately selected. Examples include 37 °C and 5% CO₂. It is preferred to change the medium at appropriate intervals during culture (preferably once per 1 day to 5 days, more preferably once per 2 days to 3 days). When the present invention process is carried out using fibroblasts as materials, brown adipocytes begin to appear in about 3 days to 7 days at 37 °C, 5% CO₂, and continue to increase for up to about 21 days to 28 days.

For culturing somatic cells, a cell culture container such as a plate, a dish, a flask for cell culture, a bag for cell culture, or the like can be used. Note that, as the bag for cell culture, one having gas permeability is suitable. Large culture vessels may be used when large amounts of cells are required. The culture can be carried out in either an open system or a closed system, but when the purpose is administration or the like of the obtained brown adipocytes to a human or the like, it is preferable to carry out the culture in a closed system.

Dexamethasone (glucocorticoid receptor agonist), insulin, 3-isobutyl-1-methylxanthine (phosphodiesterase inhibitor), triiodothyronine (thyroid hormone receptor agonist, also called T3), L-ascorbic acid 2-phosphate (ascorbic acid derivative), etc. are known to be effective agents for inducing differentiation into adipocytes. As an effective substance for inducing differentiation into brown adipocytes, for example, those commercially available as differentiation inducers can also be used. In the present invention process, it is preferable to culture somatic cells in the presence of one or more kinds, preferably three or more kinds, more preferably four or more kinds, and even more preferably five kinds of substances selected from the above-described substances. Preferably, dexamethasone, insulin, 3-isobutyl-1-methylxanthine, T3, and L-ascorbic acid 2-phosphate may be used.

In case dexamethasone is used, the concentration of dexamethasone is not particularly limited, but it can be preferably used in a range of 0.01 µmol/L to 30 µmol/L, preferably 0.1 µmol/L to 10 µmol/L.

In case insulin is used, the concentration of insulin is not particularly limited, but can be preferably used in a range of 1 ng/ml to 100 µg/ml, preferably 100 ng/ml to 20 µg/ml.

In case 3-isobutyl-1-methylxanthine is used, the concentration of 3-isobutyl-1-methylxanthine is not particularly limited, but can be preferably used in a range of 1 µmol/L to 1 mmol/L, preferably 10 µmol/L to 0.5 mmol/L.

In case T3 is used, the concentration of T3 is not particularly limited, but can be preferably used in a range of 1 nmol/L to 10 µmol/L, preferably 0.01 µmol/L to 1 µmol/L.

In case L-ascorbic acid 2-phosphate is used, the concentration of the L-ascorbic acid 2-phosphate is not particularly limited, but can be preferably used in a range of 1 µg/mL to 1 mg/mL, preferably 10 µg/mL to 250 µg/mL.

### 2 Brown adipocyte

By the above-described present invention process, a cell population containing brown adipocytes can be obtained. The brown adipocytes produced by the present invention process are also within the scope of the present invention. The brown adipocytes produced by the present invention process may not only be terminally differentiated cells but also be progenitor cells destined to differentiate into brown adipocytes.

The brown adipocytes produced by the present invention process are what is called chemical compound-induced brown adipocytes (ciBAs), which are induced to differentiate directly from somatic cells by low weight molecule compounds, and are distinguished from brown adipocytes induced to differentiate by gene transfer. Also, brown adipocytes produced by the present invention process may be beige cell-like or bright cell-like.

The brown adipocytes produced by the present invention process can be detected, confirmed, and separated using, for example, morphological changes of cells, characteristic properties of brown adipocytes, and specific markers.

Adipocytes accumulate fat within cells. Thus, adipocytes can be detected by staining of intracellular fat using Oil Red O.

Specific markers for brown adipocytes can include, but are not limited to, UCP1, EVOL3 (Elongation of very long chain fatty acid protein 3), PGC1A (PPAR gamma coactivator 1-alpha), PRDM16 (PRD1-BF1-RIZ1 homologous domain containing 16), CITED 1 (CBP/p300 interacting transactivator with Glu/Asp rich carboxy-terminal domain 1), and the like. UCP1 is a kind of uncoupling proteins.

Quarantine methods (detection with antibodies) can be used to detect specific markers, but detection can be performed with quantitation of mRNA quantities of protein molecules. Antibodies that recognize specific markers of brown adipocytes are also useful in isolating and purifying brown adipocytes obtained by the present invention process.

The brown adipocytes produced by the present invention process can be used, for example, for tissue repair or the like after surgical treatment. Brown adipocytes produced by the present invention process can be used to produce pharmaceutical compositions for tissue repair and the like. In addition, transplantation and administration of brown adipocytes to the living body are expected to have effects on the improvement of the metabolism of the living body, the prevention of obesity, and the like.

When the brown adipocytes are used as pharmaceutical compositions, they may be formulated in a form suitable for administration to an individual by conventional methods, such as by mixing brown adipocytes with a pharmaceutically acceptable carrier. Examples of the carrier can include physiological saline and distilled water for injection which is made isotonic by the addition of glucose or other auxiliary agents (e.g., D-sorbitol, D-mannitol, sodium chloride, and the like). Further, buffering agents (e.g., phosphate buffer, sodium acetate buffer), painless agents (e.g., benzalkonium chloride, procaine hydrochloride, and the like), stabilizers (e.g., human serum albumin, polyethylene glycol, and the like), preservatives, antioxidants, and the like may be formulated.

The brown adipocytes may also be formulated in compositions in combination with other cells or components effective in enhancing the functional manifestation and the engraftment of brown adipocytes.

Further, the brown adipocytes produced by the present invention process can also be used for screening pharmaceutical candidate compounds or food ingredients which acts on brown adipocytes and for evaluating the safety of pharmaceutical candidate compounds. Thus, the brown adipocytes produced by the present invention process can be used to screen for agents that enhance the glucose-lipid metabolism or heat production function of brown adipocytes. The pharmaceutical candidate compounds for the screening include, for example, existing medicines such as so-called lifestyle-related disease therapeutic drugs, metabolic disease therapeutic drugs, and other library drugs. The food ingredients include, for example, nutrients of a wide variety of foods such as vitamins, saccharides, amino acids, minerals, and the like; ingredients of functional foods; and low molecular weight compounds, proteins, lipids, and the like in Chinese medicines, herbs, or other foods.

The above-described screening can be carried out, for example, by adding a test substance to the brown adipocytes produced by the present invention process or the cells under production thereof, and evaluating the degree of increase in browning tendency in the cells by the test substance. In such evaluations, for example, the expression levels of Ucp1 gene, which is a marker specific for brown adipocytes, and Fabp4 gene, which is expressed over the whole of adipocytes, are quantified by, for example, real-time PCR, and then the ratio thereof can be used as an indicator of the tendency of browning. Pharmaceutical candidate compounds and food ingredients that enhance the browning tendency analyzed by this method can be potential candidate compounds capable of increasing brown adipocytes (beige cells) in the human body.

According to the present invention process, since a plenty of brown adipocytes can be acquired in a single operation, reproducible research results can be obtained without being affected by a lot difference in cells.

### 2 Composition

The composition for induction according to the present invention (hereinafter, referred to as the "composition of the present invention") is characterized by a composition for producing brown adipocytes by directly inducing differentiation from somatic cells, comprising a selective PPARγ agonist and a cAMP inducer, and being serum-free.

In the present invention, the composition of the present invention further comprising BMP7 is preferred.

In each of the selective PPARγ agonist and the cAMP inducer, either one kind or two or more kinds in combination may be used.

The significance and specific examples, preferred examples, and the like of the above-described selective PPARγ agonist, the cAMP inducer, and the term "serum-free" are the same as mentioned above.

The composition of the present invention can be used as a composition for producing brown adipocytes from somatic cells. The composition of the present invention can also be used as a medium for producing brown adipocytes from somatic cells.

Examples of the medium used for the production of brown adipocytes from somatic cells can include a medium in which a selective PPARγ agonist and a cAMP inducer are contained as active ingredients in a basal medium prepared by mixing components required for cell culture. The active ingredients described above may be contained in a concentration effective for producing brown adipocytes, and the concentration may be appropriately determined by one skilled in the art. The basal medium may be selected from known or commercially available media. For example, MEM (Minimum Essential Eagle's Medium), GMEM (Glasgow's Minimal Essential Medium), DMEM (Dulbecco's Modified Eagle's Medium), Ham's F-12, DMEM/F12, RPMI1640, and IMDM (Iscov's Modified Dulbecco's Medium), which are common media, can be used as a basal medium.

The medium may further be supplemented with known medium components described above herein, e.g., sera, proteins (albumin, transferrin, growth factor, etc.), amino acids, saccharides, vitamins, fatty acids, antibiotics, and the like.

The medium may further be supplemented with substances effective in inducing differentiation into adipocytes such as dexamethasone, insulin, and 3-isobutyl-1-methylxanthine, T3, L-ascorbic acid 2-phosphate, as described herein above.

### EXAMPLES

Hereinafter, the present invention will be specifically illustrated by way of Examples, Comparative Examples, and Test Examples, but the present invention is not limited to the scope of the Examples and the like.

### [Example] Production of Brown Adipocytes

### (1) Cell culture

The human fibroblasts used as the material were purchased from DS Pharma Biomedical Co., Ltd. The fibroblasts are derived from 38-year-old human skin.

The above human fibroblasts were seeded in 35 mm dishes coated with gelatin (Cat#: 190-15805, manufactured by FUJIFILM Wako Pure Chemical Co.) at 5×10⁴ , and cultured in DMEM medium (Cat#: 11965092; manufactured by Gibco) supplemented with 10% fetal bovine serum (FBS), 100 U/mL penicillin, 100 µg/mL streptomycin until 80% to 90% confluency at 37 °C, 5% CO₂.

### (2) Direct conversion of human fibroblasts to brown adipocytes (direct differentiation induction)

The serum-free medium (serum-free brown adipocyte-inducing medium) used for direct conversion into brown adipocytes was prepared by adding each component of Table 1 to a DMEM medium (Cat#: 11965092; manufactured by Gibco), and further adding each compound shown in Table 2 so as to have the said final concentration, in combinations of the compounds shown in Table 3. Hereinafter, this is referred to as serum-free brown adipocyte-inducing medium "SFBAM".

**[Table 1]**

| Ingredients | Final conc. |
|---|---|
| Triiodothyronine (T6397, by Merck) | 0.1 µmol/L |
| Dexamethasone (041-18863, by FUJIFILM Wako Pure Chemical Co.) | 0.5 µmol/L |
| IBMX (3-isobutyl-1-methylxanthine) (095-03413, by the same company above) | 30 µmol/L |
| Insulin (093-06351, by the same company above) | 6.6 µg/mL |
| L-ascorbic acid 2-phosphate (323-44822, by the same company above) | 100 µg/mL |
| Linoleic acid-Oleic acid-Albumin (L9655, by Merck) | 1 mg/mL |
| Penicillin-Streptomycin (15070063, by Gibco) | 100 U/mL |

**Table 2]**

| Compounds | Concentration | |
|---|---|---|
| SB431542 (198-16543, by FUJIFILM Wako Pure Chemical Co.) | 2 µmol/L | ALK5 inhibitor |
| LDN193189 (124-06011, by the same company above) | 1 µmol/L | ALK2/3 inhibitor |
| Dorsomorphine (040-33753, by the same company above) | 1 µmοl/L | ALK2/3/6 inhibitor |
| Rosiglitazone (184-02651, by the same company above) | 1 µmol/L | Selective PPARγ agonist |
| Forskolin (063-02193, by the same company above) | 7.5 µmol/L | cAMP inducer |
| BMP7 (026-19171, by the same company above) | 20 ng/mL | Osteogenic protein |

**[Table 3]**

| Compounds | Example 1 | Example 2 | Example 3 | Example 4 | Comp. Ex. 1 | Comp. Ex. 2 | Comp. Ex. 3 |
|---|---|---|---|---|---|---|---|
| Rosiglitazone | + | + | + | + | + | - | - |
| Forskolin | + | + | + | + | - | + | - |
| BMP7 | - | + | + | - | + | + | - |
| LDN193189 | + | - | - | - | - | - | - |
| Dorsomorphine | + | - | - | - | - | - | - |
| SB431542 | + | + | - | - | - | - | - |

In Table 3, the mark "+" indicates the presence of the compound in the medium, and the mark "-" indicates the absence of the compound in the medium.

After the human fibroblasts reached 80% to 90% confluency, the dish medium was replaced with the above SFBAM and the human fibroblasts were cultured in SFBAM for 3 weeks. The medium was changed every 3 days. Note that, for example, in a particular experiment according to Figure 9, the chemically induced brown adipocyte-like cells (ciBAs) were made to mature by further culturing for 1 week in a serum-free brown adipocyte maintenance medium (hereinafter referred to as "SFBAMaM") prepared by adding the respective components shown in Table 4 to a DMEM medium (Cat#: 11965092; manufactured by Gibco).

**[Table 4]**

| Compounds | Final conc. |
|---|---|
| Rosiglitazone (184-02651, by FUJIFILM Wako Pure Chemical Co.) | 1 µmol/L |
| Dexamethasone (041-18863, by the same company above) | 0.5 µmol/L |
| Insulin (093-06351, by the same company above) | 6.6 µg/mL |
| Linoleic acid-Oleic acid-Albumin (L9655, by Merck) | 1 mg/mL |
| Penicillin-Streptomycin (15070063, by Gibco) | 100 U/mL |

### (3) Immunostaining

Immunocytochemistry was performed as previously described (Dai P, et al. J Clin Biochem Nutr. 2015; 56(3): 166-70). First, the cells were fixed with 4% paraformaldehyde (Nacalai Tesque). After washed with phosphate-buffered saline (PBS) three times, the cells were incubated with PBS containing 0.1% Triton X-100 for 10 min. The cells were then blocked with PBS containing 3% skim milk for 1 hour at room temperature. UCP-1 antibodies (ab10983, Abcam, Cambridge, UK) were diluted 1/500 with blocking solutions. The cells were incubated with the antibodies overnight at 4 °C. After washed with PBS three times, the cells were incubated with Alexa Fluor 488 Donkey anti-Rabbit IgG (A-21206, Thermo Fisher Scientific) for 1 hour at room temperature. Cell nuclei were stained with DAPI solutions (Dojindo Laboratories). All images were acquired using fluorescent microscopy (Axio Vert.A1, Carl Zeiss, Oberkochen, Germany). Mitochondria were stained using MitoTracker (registered trademark) Red CMXRos (Thermo Fisher Scientific).

### (4) RNA isolation and QRT-PCR

To quantify gene expression, total RNAs were extracted from fibroblasts treated with the respective compounds using FastGene (registered trademark) RNA basic kit (manufactured by Nippon Genetics Co., Ltd.). Fibroblasts were cultured in parallel using SFBAM as a control, and the total RNAs were extracted from the brown adipocytes induced with compounds and the control cells. After performing reverse-transcription with ReverTraAce (registered trademark) PCR RT Master Mix with gDNA Remover (manufactured by Toyobo Co., Ltd.), the real-time PCR analysis was performed by Power SYBR Green PCR Master Mix (Thermo Fisher Scientific). Three reactions were carried out per level under the following conditions: 40 cycles of reactions at 95°C for 10 minutes, at 95°C for 15 seconds, and at 60 °C for 60 seconds, in order. All results were normalized to the quantity of Tbp mRNA. Primers used in QRT-PCR are shown in Table 5 below.

**[Table 5]**

| Genes | Sense primer | Antisense primer |
|---|---|---|
| *Tbp* | ACTACGGGGTTATCACCTGTGAG (Sequence No.1) | GTGCAGGAGTAGGCCACATTAC (Sequence No.2) |
| *Ucp1* | TCTACGACACGGTCCAGGAG (Sequence No.3) | GAATACTGCCACTCCTCCAGTC (Sequence No.4) |
| *Fabp4* | GCCAGGAATTTGACGAAGTCA (Sequence No.5) | CCCATTTCTGCACATGTACCAG (Sequence No.6) |

### (5) Results

The experimental results are shown in the drawings. Note that throughout the figures, the data indicate the mean ± SD (n=3), the marks "^{∗∗∗}", "^{∗∗}" and "^{∗}" indicate significant differences at p<0.001, p<0.01 and p<0.05, respectively, by Student t-test, and the mark "N.S." indicates no significant differences.

As shown in Figure 1, the combination of compounds including rosiglitazone (selective PPARγ agonist) and forskolin (cAMP inducer) provides sufficient direct differentiation induction (direct conversion) from human fibroblasts into brown adipocytes in serum-free medium SFBAM. In addition, as shown in Figures 1 and 2, the combination of Example 2, in which LDN193189 (ALK2/3 inhibitor) and the dorsomorphin (ALK2/3/6 inhibitor) were removed from and BMP7 was added to the combination of the compounds of Example 1, improved the conversion efficiency into UCP1 positive cells, that is, brown adipocytes, as compared with the combination of the compounds of Example 1, in the serum-free medium.

As shown in Figure 3, in the serum-free medium, the expression of Fabp4, an adipocyte-specific gene, was elevated compared to the control (Comparative Example 3, no compound), but no significant difference was observed between the cases of Example 1 and Example 2 (Figure 3B). However, the expression level and the rate of Ucp1, a brown adipocyte-specific gene, was greatly increased in the combination of the compounds of Example 2 as compared to the combination of the compounds of Example 1 (Figure 3A). In addition, the ratio of the expression amount of Ucp1 to the expression amount of Fabp4 was greatly increased in the case of Example 2 as compared to Example 1 (Figure 3C). Also, an increase in UCP1 proteins was observed in Example 2 as compared to Example 1, corresponding to an increase in the expression level of Ucp1 mRNA (Figure 3D).

As shown in Figure 4, in the serum-free medium, the combination of the compounds of Example 3 (forskolin, rosiglitazone and BMP7), in which SB431542 (ALK5 inhibitor) was removed from the combination of the compounds of Example 2, further increased the expression of Ucp1, a brown adipocyte-specific gene. Correspondingly, the quantity of UCP1 proteins was also increased in the case of Example 3.

The result in the case that either one compound was removed from the combination of the compounds of Example 3 (forskolin, rosiglitazone and BMP7) is shown in Figure 5. As shown in Figure 5, in the serum-free medium, when the rosiglitazone (selective PPARγ agonist) was removed from the combination of the compounds of Example 3 (Symbol 3, Comparative Example 2), direct conversion into brown adipocytes was scarcely observed. On the other hand, even if forskolin (cAMP inducer) was removed from the combination of the compounds of Example 3 (Symbol 4, Comparative Example 1), or BMP7 was removed (Symbol 5, Example 4), direct conversion into brown adipocytes was observed, albeit only a little, and direct conversion was harder to occur when forskolin was removed.

Thus, in the direct conversion of human fibroblasts to brown adipocytes in the serum-free medium, it is considered that rosiglitazone (selective PPARγ agonist) has a high conversion efficiency, followed by a high conversion efficiency of forskolin (cAMP inducer).

For each combination of compounds, the direct conversion efficiency to adipocyte-like cells in the serum-free medium was evaluated from the results shown in Figures 1 to 5. The results are shown in Table 6 below. Note that Figure 1 shows the results of immunostaining observed with a phase contrast microscope, and the efficiency was judged comprehensively based on the state of the cells. The more the number of + marks, the higher the conversion efficiency. For example, the mark ++++ indicates that most of the cells converted to adipocytes were the cells which had been converted to UCP1 positive brown adipocytes. The mark - indicates no conversion to brown adipocytes.

**[Table 6]**

| Combinations | Efficiency |
|---|---|
| Example 1 | + + |
| Example 2 | + + + |
| Example 3 | + + + + |
| Example 4 | + + + or + + |
| Comp. Ex. 1 | + |
| Comp. Ex. 2 | - |
| Comp. Ex. 3 | - |

### [Test Example 1] Direct Conversion of Different Human Fibroblasts to Brown Adipocytes

Three distinct human dermal fibroblasts were cultured for three weeks in a serum-free inducing medium SFBAM supplemented with the compounds of Example 3 (rosiglitazone, forskolin and BMP7) in the same manner as in Examples (1) and (2) above. Several specific marker-genes for adipocytes and brown adipose tissue were then quantified by QRT-PCR.

Human skin fibroblasts subjected to the experiment were purchased from DS Pharma Biomedical Corporation. The cell information is shown in Table 7 below.

**[Table 7]**

| | Lot # | Passage No. | BMI | Age | Gender | Depot |
|---|---|---|---|---|---|---|
| HDF22 | DFM062509 | 3 | 29 | 22 | Man | Chest |
| HDF38 | DFM090214 | 3 | 23.1 | 38 | Man | Abdomen |
| HDF54 | DDFM052010B | 3 | 21.3 | 54 | Woman | Abdomen |

Figure 6 shows these results. Ucp1 mRNA was significantly induced in all fibroblasts in serum-free medium compared to the control without the addition of the compounds (Comparative Example 3) (Figure 6A). In brown adipocytes induced with the compounds in serum-free medium, the expression of Fabp4, an adipogenic differentiation marker, was increased for all fibroblasts (Figure 6B).

Immunocytochemical analyses were also performed to analyze mitochondrial multiplication, expression of UCP1 proteins, and conversion efficiencies to brown adipocytes (Figures 7 and 8). Consequently, the majority of adipocytes were found to be positive for UCP1, indicating that they have characteristics of brown adipocytes. In addition, UCP1 stain was well overlapped with the increase in mitochondrial signal.

### [Test Example 2] Study of Heat Production Capacity of Brown Adipocytes Induced to Differentiate by the Present Invention

Thermogenesis via UCP1 is regulated by the sympathetic nervous system and the β-adrenergic receptor signal pathway in brown adipocytes. To evaluate the thermogenic ability of the brown adipocytes induced to differentiate with the present invention, human fibroblasts were directly converted by the combination of the compounds of Example 3 for 3 weeks in the serum-free medium SFBAM, followed by being cultured for another 1 week in the serum-free medium SFBAMaM to make the directly converted cells mature, in the same manner as in the above Examples (1) and (2). Regarding the cells, Ucp1 mRNA was quantified in the case after treatment with isoproterenol (β-adrenergic receptor agonist) for 6 hours and in the case after treatment with H₂O, which is the solvent. The results are shown in Figure 9.

As shown in Figure 9, compared with the control treated with H₂O, Ucp1 mRNA after treatment with isoproterenol was markedly increased by up to 3.1-fold.

Thus, in the serum-free medium, brown adipocytes induced to differentiate with the combination of the compounds of Example 3, i.e., brown adipocytes induced to differentiate from fibroblasts by the present invention, are shown to be capable of responding to the β-adrenergic receptor signaling pathway for thermogenic gene induction.

### [Test Example 3] Measurement of Oxygen Consumption Rate of Brown Adipocytes Induced to Differentiate by the Present Invention

To measure the rate of oxygen consumption by mitochondria (oxygen consumption rate: OCR), human fibroblasts were directly converted by the combination of the compounds of Example 3 (rosiglitazone, forskolin and BMP7) in the serum-free medium SFBAM in 96 well plates for 3 weeks, in the same manner as in the above Example (1) and (2). As a control, human fibroblasts were cultured in parallel in the serum-free medium SFBAM. After incubation in a non-CO₂ incubator at 37 °C for 1 hour, the oxygen-consumption rate of each well containing brown adipocytes induced with the compounds was analyzed using XF96 Extracellular Flux Analyzer (Seahorse Bioscience Inc., Billerica, MA) according to the instructions. During the analyses, the mitochondrial electron-transport system inhibitors oligomine, FCCP (carbonylcyanide 4-(trifluoromethoxy) phenylhydrazone), and antimycin A/rotenone were added to each well via an injection device to final concentrations of 2 µM, 0.3 µM, and 0.5 µM, respectively.

The results are shown in Figure 10. As shown in Figure 10A, OCR values were typically altered by adding mitochondrial inhibitors during the analyses, and brown adipocytes directly converted from human fibroblasts by the combination of the compounds of Example 3 showed higher OCR compared to the control. As shown in Figure 10B, the OCR values corresponding to the mitochondrial activities were also increased compared to the control, respectively.

Thus, it is clear that brown adipocytes induced to differentiate by the combination of the compounds of Example 3, i.e., brown adipocytes induced to differentiate from fibroblasts by the present invention, in the serum-free medium, have an increased oxidative metabolism in mitochondria.

### [Test Example 4] Investigation of induction period to brown adipocytes.

Human skin fibroblasts were cultured in the serum-free inducing medium SFBAM supplemented with the compounds of Example 3 (rosiglitazone, forskolin and BMP7), in the same manner as in the above Examples (1) and (2). Then, the expression levels of a plurality of marker genes shown in Table 8 below, which are specific for adipocytes and brown adipose tissue, were quantified by QRT-PCR. The results are shown in Figure 11. In the figures, the mark "C" shows the case of culturing in the same manner for 5 weeks without the compounds of Example 3, the mark "Ro" shows the case of culturing in the same manner for 5 weeks with only rosiglitazone among the compounds of Example 3. Note that the genes shown in the figure 11A are specific to brown adipocytes, and the genes shown in the figure 11B are specific to adipocytes in general.

**[Table 8]**

| Genes | Sense primer | Antisense primer |
|---|---|---|
| *Tbp* | ACTACGGGGTTATCACCTGTGAG (Sequence No.1) | GTGCAGGAGTAGGCCACATTAC (Sequence No.2) |
| *Ucp1* | TCTACGACACGGTCCAGGAG (Sequence No.3) | GAATACTGCCACTCCTCCAGTC (Sequence No.4) |
| *Fabp4* | GCCAGGAATTTGACGAAGTCA (Sequence No.5) | CCCATTTCTGCACATGTACCAG (Sequence No.6) |
| *Ckmtl* | AGCAGGAATGGCTCGAGAC (Sequence No.7) | ATCCTCCTCATTCACCCAGATC (Sequence No.8) |
| *Cited1* | TGGCACCTCACCTGCGAAG (Sequence No.9) | GCAGAATGGCCACTGCTTTG (Sequence No.10) |
| *AdipoQ* | CTGGTGAGAAGGGTGAGAAAG (Sequence No.11) | GTTTCACCGATGTCTCCCTTAG (Sequence No.12) |

As shown in Figure 11, both genes started to be expressed by the first week of culture, and the expression levels continued to increase until the third to fourth week. It also seems from this result that brown adipocytes induced to differentiate from fibroblasts by the present invention begin to appear by week 1 and continue to increase until week 3 or week 4.

## Claims

1. A process for producing brown adipocytes by direct differentiation induction from somatic cells, comprising a step of culturing a somatic cell in a serum-free differentiation induction medium in the presence of a selective PPARγ agonist and a cAMP inducer.

2. The process for producing brown adipocytes according to Claim 1, wherein the step is a step of culturing a somatic cell in the further presence of BMP7

3. The process for producing brown adipocytes according to Claim 1 or 2, wherein the step is a step of culturing a somatic cell in the absence of an ALK2/3 inhibitor and an ALK6 inhibitor.

4. The process for producing brown adipocytes according to any one of Claims 1 to 3, wherein the step is a step of culturing a somatic cell in the further absence of an ALK5 inhibitor.

5. The process for producing brown adipocytes according to any one of Claims 1 to 4, wherein the selective PPARγ agonist is rosiglitazone or the cAMP inducer is forskolin.

6. The process for producing brown adipocytes according to any one of Claims 1 to 5, wherein the somatic cell is a fibroblast.

7. A brown adipocyte, produced by the process according to any one of Claims 1 to 6.

8. A differentiation induction composition for producing brown adipocytes by directly inducing differentiation from somatic cells, comprising a selective PPARγ agonist and a cAMP inducer and being serum-free.

9. The differentiation induction composition according to Claim 8, further comprising BMP7.

10. The differentiation induction composition according to Claim 8 or 9, wherein the selective PPARγ agonist is rosiglitazone or the cAMP inducer is forskolin.

11. The differentiation induction composition according to any one of Claims 8 to 10, wherein the somatic cell is a fibroblast.

12. A method of screening an agent acting on brown adipocytes, comprising using the brown adipocyte produced by the process according to any one of Claims 1 to 6, or the brown adipocyte according to Claim 7.

13. A method of evaluating the degree of enhancement of browning tendency by a test substance in the brown adipocyte produced by the process according to any one of Claims 1 to 6 or the cell under production thereof, or the brown adipocyte according to Claim 7, comprising an addition of the test substance to the cells.


SEQUENCE LISTING

<110>  KATAOKA CORPORATION
       Kyoto Prefectural Public University Corporation

<120>  A method for producing a brown adipocyte

<130>\201@P19028WO

<160>  12

<170>  PatentIn version 3.5

<210>  1
<211>  23
<212>  DNA
<213>  Artificial Sequence
<220>
<223>  Description of Artificial Sequence: Synthetic DNA
<400>  1
actacggggt tatcacctgt gag                                              23

<210>  2
<211>  22
<212>  DNA
<213>  Artificial Sequence
<220>
<223>  Description of Artificial Sequence: Synthetic DNA
<400>  2
gtgcaggagt aggccacatt ac                                               22

<210>  3
<211>  20
<212>  DNA
<213>  Artificial Sequence
<220>
<223>  Description of Artificial Sequence: Synthetic DNA
<400>  3
tctacgacac ggtccaggag                                                  20

<210>  4
<211>  22
<212>  DNA
<213>  Artificial Sequence
<220>
<223>  Description of Artificial Sequence: Synthetic DNA
<400>  4
gaatactgcc actcctccag tc                                               22

<210>  5
<211>  21
<212>  DNA
<213>  Artificial Sequence
<220>
<223>  Description of Artificial Sequence: Synthetic DNA
<400>  5
gccaggaatt tgacgaagtc a                                                21

<210>  6
<211>  22
<212>  DNA
<213>  Artificial Sequence
<220>
<223>  Description of Artificial Sequence: Synthetic DNA
<400>  6
cccatttctg cacatgtacc ag                                                22

<210>  7
<211>  19
<212>  DNA
<213>  Artificial Sequence
<220>
<223>  Description of Artificial Sequence: Synthetic DNA
<400>  7
agcaggaatg gctcgagac                                                    19

<210>  8
<211>  22
<212>  DNA
<213>  Artificial Sequence
<220>
<223>  Description of Artificial Sequence: Synthetic DNA
<400>  8
atcctcctca ttcacccaga tc                                                22

<210>  9
<211>  19
<212>  DNA
<213>  Artificial Sequence
<220>
<223>  Description of Artificial Sequence: Synthetic DNA
<400>  9
tggcacctca cctgcgaag                                                    19

<210>  10
<211>  20
<212>  DNA
<213>  Artificial Sequence
<220>
<223>  Description of Artificial Sequence: Synthetic DNA
<400>  10
gcagaatggc cactgctttg                                                   20

<210>  11
<211>  21
<212>  DNA
<213>  Artificial Sequence
<220>
<223>  Description of Artificial Sequence: Synthetic DNA
<400>  11
ctggtgagaa gggtgagaaa g                                                 21

<210>  12
<211>  22
<212>  DNA

<213>  Artificial Sequence
<220>
<223>  Description of Artificial Sequence: Synthetic DNA
<400>  12
gtttcaccga tgtctccctt ag                                                22